(19) **Europäisches Patentamt / European Patent Office / Office européen des brevets**

(11) **EP 3 777 668 B1**

(12) # EUROPEAN PATENT SPECIFICATION

(45) Date of publication and mention of the grant of the patent:
**27.03.2024 Bulletin 2024/13**

(21) Application number: **19784486.3**

(22) Date of filing: **10.04.2019**

(51) International Patent Classification (IPC):
$A61B\ 5/0245^{(2006.01)}$    $A61B\ 5/11^{(2006.01)}$
$A61B\ 5/355^{(2021.01)}$    $A61B\ 5/352^{(2021.01)}$
$A61B\ 5/36^{(2021.01)}$    $A61B\ 5/22^{(2006.01)}$
$A61B\ 5/024^{(2006.01)}$    $A63B\ 24/00^{(2006.01)}$
$A61B\ 5/00^{(2006.01)}$

(52) Cooperative Patent Classification (CPC):
**A61B 5/02405; A61B 5/1118; A61B 5/222;
A61B 5/352; A61B 5/355; A61B 5/36;**
A61B 5/0245; A61B 5/329; A61B 5/4035;
A61B 2503/10

(86) International application number:
**PCT/JP2019/015579**

(87) International publication number:
**WO 2019/198744 (17.10.2019 Gazette 2019/42)**

(54) **MAXIMUM HEART RATE ESTIMATION METHOD AND DEVICE**

VERFAHREN UND VORRICHTUNG ZUR SCHÄTZUNG DER MAXIMALEN HERZFREQUENZ

PROCÉDÉ ET DISPOSITIF D'ESTIMATION DE FRÉQUENCE CARDIAQUE MAXIMALE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **12.04.2018 JP 2018076550**

(43) Date of publication of application:
**17.02.2021 Bulletin 2021/07**

(73) Proprietor: **NIPPON TELEGRAPH AND
TELEPHONE CORPORATION
Chiyoda-ku, Tokyo 100-8116, (JP)**

(72) Inventors:
• **HIGUCHI, Yuichi**
  **Musashino-shi, Tokyo 180-8585 (JP)**
• **TOGO, Hiroyoshi**
  **Musashino-shi, Tokyo 180-8585 (JP)**
• **MATSUURA, Nobuaki**
  **Musashino-shi, Tokyo 180-8585 (JP)**

(74) Representative: **Samson & Partner Patentanwälte
mbB
Widenmayerstraße 6
80538 München (DE)**

(56) References cited:
JP-A- 2004 000 646    US-A- 4 622 980
US-A1- 2004 147 850    US-A1- 2017 172 513
US-B1- 8 755 872

• LAURA KARAVIRTA ET AL: "Estimation of
maximal heart rate using the relationship
between heart rate variability and exercise
intensity in 40-67 years old men", EUROPEAN
JOURNAL OF APPLIED PHYSIOLOGY,
SPRINGER, BERLIN, DE, vol. 103, no. 1, 11
January 2008 (2008-01-11), pages 25-32,
XP019590023, ISSN: 1439-6327
• Motohisa Osaka: "Heart rate fluctuations and
autonomic nerves", Journal of Nippon Medical
School, vol. 62, no. 1, 15 February 1995
(1995-02-15), pages 71-75, XP055741723, ISSN:
0048-0444, DOI: 10.1272/jnms1923.62.71

EP 3 777 668 B1

**Description**

Technical Field

The present invention relates to a maximum heart rate estimation method and device and, more particularly, to a technique of estimating a maximum heart rate using an electrocardiographic waveform.

Background Art

In sports and daily life, it is possible to estimate exercise intensity and calories by managing a heart rate. Exercise intensity using a heart rate can generally be calculated by the Karvonen method (see, for example, non-patent literature 1). Therefore, to calculate exercise intensity, it is necessary to measure or estimate the maximum heart rate. Furthermore, the value of exercise intensity is necessary to calculate calories, and it is thus important to measure or estimate the maximum heart rate.

Conventionally, when measuring the maximum heart rate, measurement is performed by increasing momentum until the heart rate of a target person reaches the maximum heart rate by an incremental load test or the like.

Related Art Literature

LAURA KARAVIRTA ET AL. "Estimation of maximal heart rate using the relationship between heart rate variability and exercise intensity' in 40-67 years old men" (European Journal of Applied Physiology, vol. 103, no. 1, pages 25-32) discloses subject-matter according to the preamble of claim 1.

US 2004/147850 A1 relates to an exercise load intensity evaluation device and exercise equipment.

US 8 755 872 B1 relates to a patient monitoring system for indicating an abnormal condition.

US 2017/172513 A1 relates to a method and system for anaerobic threshold heart rate detection.

US 4 622 980 A relates to a method and apparatus for determining of stress condition of a subject.

Non-Patent Literature

Non-Patent Literature 1: Jinhua She, Hitoshi Nakamura, Koji Makino, Hiroshi Hashimoto, "Selection of Suitable Maximum-heart-rate Formulas for Use with Karvonen Formula to Calculate Exercise Intensity", International Journal of Automation and Computing, 12(1), February 2015, pp. 62 - 69.
Non-Patent Literature 2:

https://ja.wikipedia.org/wiki/運動強度 (searched on March 20, 2018)
Non-Patent Literature 3: Shaffer, Fred, and J.P.Ginsberg, "An overview of heart rate variability metrics and norms." Frontiers in public health 5 (2017): 258
Non-Patent Literature 4: Hideaki Senju, "Chapter 3 Exercise and Change in Cardiopulmonary Function (Part I Regional Wellness)" Health Promotion from Life/Region, Nagasaki University Extension Course Series 7 (published on March 5, 1995): pp. 31 - 39, <http://hdl.handle.net/10069/6315>

Disclosure of Invention

Problem to be Solved by the Invention

However, in the conventional method of measuring the maximum heart rate by an incremental load test or the like, the load of a target person is heavy.

The present invention has been made in consideration of the above problem, and has as its object to provide a maximum heart rate estimation method and device capable of estimating the maximum heart rate without requiring exercise to be done until the heart rate of a target person reaches the maximum heart rate. Means of Solution to the Problem

The present invention solves the above-described problem by the maximum heart rate estimation method according to claim 1 and the maximum heart rate estimation device according to claim 2.

Effect of the Invention

According to the present invention, since the maximum heart rate is calculated from the relationship between the heart rate of a target person and a predetermined feature amount included in the electrocardiographic waveform of the target person based on an inflection point in a change of the feature amount with respect to the heart rate, it is possible to estimate the maximum heart rate without requiring exercise to be done until the heart rate of the target person reaches the maximum heart rate. Therefore, it is possible to reduce the load of the target person when obtaining the maximum heart rate.

Brief Description of Drawings

Fig. 1 is a view for explaining the principles of the present invention;
Fig. 2 is a graph for explaining the principles of a maximum heart rate estimation device according to the first embodiment of the present invention;
Fig. 3 is a graph for explaining the principles of the maximum heart rate estimation device according to the first embodiment of the present invention;
Fig. 4 is a block diagram showing the functional arrangement of the maximum heart rate estimation device according to the first embodiment of the present invention;
Fig. 5 is a block diagram showing the hardware arrangement of the maximum heart rate estimation device according to the first embodiment of the present invention;
Fig. 6 is a flowchart for explaining a maximum heart rate estimation method according to the first embodiment of the present invention;
Fig. 7 is a graph for explaining the principles of a maximum heart rate estimation device according to the second embodiment of the present invention;
Fig. 8 is a block diagram showing the functional arrangement of the maximum heart rate estimation device according to the second embodiment of the present invention;
Fig. 9 is a flowchart for explaining a maximum heart rate estimation method according to the second embodiment of the present invention;
Fig. 10 is a graph for explaining the principles of a maximum heart rate estimation device according to the third embodiment of the present invention;
Fig. 11 is a block diagram showing the functional arrangement of the maximum heart rate estimation device according to the third embodiment of the present invention; and
Fig. 12 is a flowchart for explaining a maximum heart rate estimation method according to the third embodiment of the present invention.

Best Mode for Carrying Out the Invention

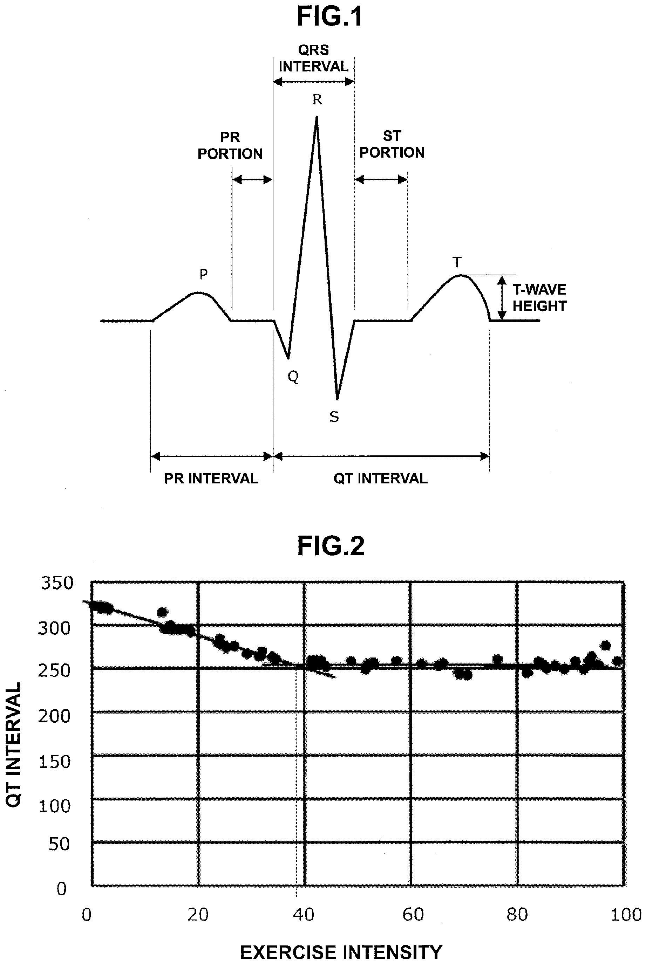

Preferred embodiments of the present invention will be described in detail below with reference to Figs. 1 to 12.

[Principles of Invention]

Fig. 1 is a view showing an electrocardiographic waveform. A maximum heart rate estimation method according to an embodiment of the present invention extracts a predetermined feature amount serving as an index of the maximum heart rate from the electrocardiographic waveform of a target person, and estimates, for example, the maximum heart rate for obtaining exercise intensity. The exercise intensity is a scale representing the vigorousness of exercise with reference to the physical ability of the target person who does the exercise.

In the maximum heart rate estimation method according to this embodiment, among characteristic waveforms and waveform intervals included in the electrocardiographic waveform shown in Fig. 1, a QT interval representing the time from the start of a Q wave to the end of a T wave is used as a feature amount of the electrocardiographic waveform (electrocardiogram).

Fig. 2 is a graph showing the relationship between a QT interval and exercise intensity acquired by an incremental load test. In this incremental load test, as the exercise intensity, a value calculated from a heart rate (HR) is used. As shown in Fig. 2, it is apparent that there is an inflection point in the graph around a portion where the exercise intensity is 40%. This experiment indicates that a heart rate at the inflection point corresponds to exercise intensity of about 40%.

This experiment result is considered to coincide with the relationship between a change in cardiac output and a stroke volume by exercise described in non-patent literature 4. In general, the oxygen demand of a peripheral tissue such as a muscle is increased by exercise. As described in non-patent literature 4, it is known that the stroke volume (SV) as a blood volume ejected by one contraction of the heart increases at a predetermined rate along with an increase in exercise intensity but reaches a plateau without increasing after the exercise intensity increases to about 40% of the maximum exercise intensity. Therefore, in exercise in which the exercise intensity exceeds about 40%, the cardiac output (CO) as a blood volume ejected from the heart per minute is supplemented by increasing the heart rate.

From this viewpoint, it is considered that it is possible to estimate the maximum heart rate using the above-described experiment result by setting the heart rate at the inflection point of the QT interval to correspond to exercise intensity of 40%.

At this time, the maximum heart rate is calculated (see, for example, non-patent literature 2) by:

$$\text{maximum heart rate} = \text{resting heart rate} + 100$$
$$\times \ (\text{heart rate at inflection point} - \text{resting heart}$$
$$\text{rate})/\text{exercise intensity at inflection point} \qquad ...(1)$$

In equation (1) above, "resting heart rate" is a value measured in advance at the time of rest by a heart rate meter or the like. In general, 60 bpm is used as the resting heart rate. "Heart rate at inflection point" is the value of a measured heart rate corresponding to the inflection point of the QT interval. In addition, "exercise intensity at inflection point" is a preset value, and a value falling within the range of 35% to 45%, for example, 40% is used based on the above-described experiment result.

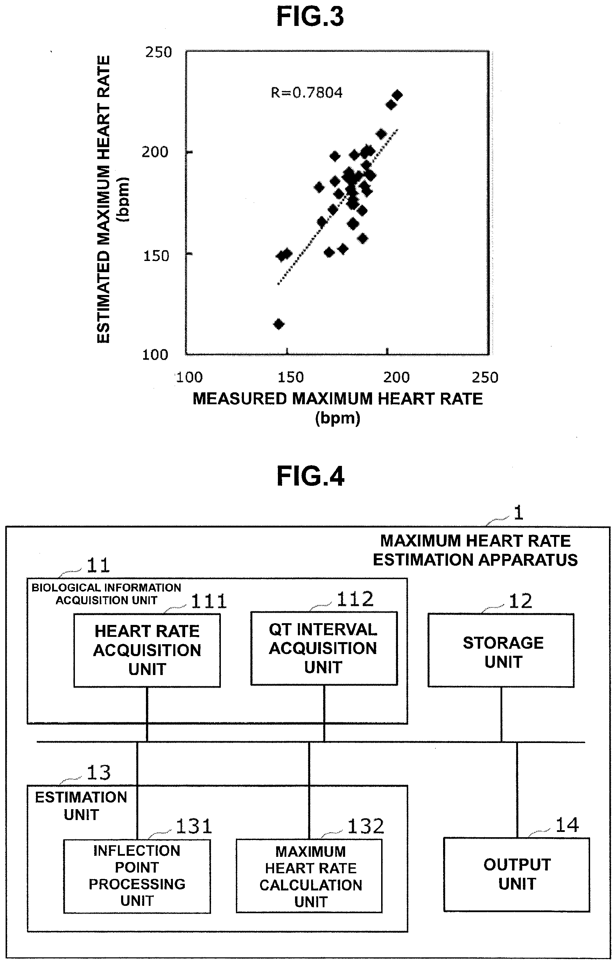

Fig. 3 is a graph showing a result when estimating the maximum heart rate using equation (1) above. In Fig. 3, the ordinate represents the maximum heart rate estimated by the maximum heart rate estimation method according to this embodiment and the abscissa represents the maximum heart rate measured by doing exercise until the heart rate of the target person reaches the maximum heart rate.

As shown in Fig. 3, since a correlation coefficient (Pearson correlation function) is 0.78 and p value < 0.05 is satisfied, it is found that there is a significant positive correlation. Therefore, the maximum heart rate estimation method according to this embodiment finds the inflection point of the QT interval in the relationship between the QT interval and the heart rate, and calculates the maximum heart rate using equation (1) above.

[First Embodiment]

A maximum heart rate estimation device 1 for executing a maximum heart rate estimation method according to the present invention will be described in detail below.

Fig. 4 is a block diagram showing the functional arrangement of the maximum heart rate estimation device 1 according to the first embodiment. The maximum heart rate estimation device 1 includes a biological information acquisition unit 11, a storage unit 12, an estimation unit 13, and an output unit 14.

The maximum heart rate estimation device 1 acquires the relationship between a QT interval and a heart rate when a target person does exercise like an incremental load test that gradually increases exercise intensity, extracts the inflection point of the value of QT interval, and calculates the maximum heart rate of the target person from the inflection point.

The biological information acquisition unit 11 includes a heart rate acquisition unit 111 and a QT interval acquisition unit 112.

The biological information acquisition unit 11 acquires information concerning the heart beats and electrocardiogram of the target person from an external biological sensor (not shown) having the functions of the heart rate meter and electrocardiograph and attached to the target person. At this time, the biological information acquisition unit 11 acquires, from the above-described experiment result, information concerning the heartbeats and electrocardiogram for a period from when the target person starts exercise that gradually increases the exercise intensity until the exercise intensity exceeds 40%.

The heart rate acquisition unit 111 acquires, from the biological sensor attached to the target person, a heart rate for a period during which the target person does exercise. Data of the acquired heart rate is stored in the storage unit 12.

The QT interval acquisition unit 112 acquires a QT interval included in the electrocardiographic waveform from the electrocardiographic waveform measured by the biological sensor attached to the target person. The QT interval acquired by the QT interval acquisition unit 112 is stored in the storage unit 12.

The storage unit 12 stores data of the heart rate and the QT interval of the target person acquired by the biological information acquisition unit 11. Furthermore, the storage unit 12 stores setting values of "resting heart rate" and "exercise intensity at inflection point" in equation (1) above.

The estimation unit 13 includes an inflection point processing unit 131 and a maximum heart rate calculation unit 132.

The estimation unit 13 estimates the maximum heart rate based on the data of the heart rate and the QT interval of the target person acquired by the biological information acquisition unit 11.

The inflection point processing unit 131 reads out, from the storage unit 12, the data of the heart rate of the target person acquired by the heart rate acquisition unit 111 and the data of and the QT interval of the target person acquired by the QT interval acquisition unit 112, and obtains the relationship between the QT interval and the heart rate. At this time, the graph including the inflection point shown in Fig. 2 is obtained.

Furthermore, the inflection point processing unit 131 extracts, from the relationship between the QT interval and the heart rate of the target person, an inflection point in a change of the QT interval with respect to the heart rate acquired by the heart rate acquisition unit 111. The inflection point processing unit 131 obtains the heart rate of the target person corresponding to the inflection point of the value of QT interval, and stores it in the storage unit 12.

The maximum heart rate calculation unit 132 calculates the maximum heart rate of the target person by equation (1) above based on the heart rate of the target person at the inflection point extracted by the inflection point processing unit 131.

More specifically, the maximum heart rate calculation unit 132 substitutes, into equation (1), the value of "heart rate at inflection point" obtained by the inflection point processing unit 131. Note that in equation (1), a preset value, for example, 60 bpm is used as the value of "resting heart rate" and a preset value, for example, 40% is used as the value of "exercise intensity at inflection point".

The maximum heart rate calculation unit 132 stores the calculated value of the maximum heart rate of the target person in the storage unit 12.

The output unit 14 outputs information such as the maximum heart rate of the target person estimated by the estimation unit 13. More specifically, the output unit 14 displays the value of the maximum heart rate calculated by the maximum heart rate calculation unit 132 on a display screen or the like.

[Hardware Arrangement of Maximum Heart Rate Estimation Device]

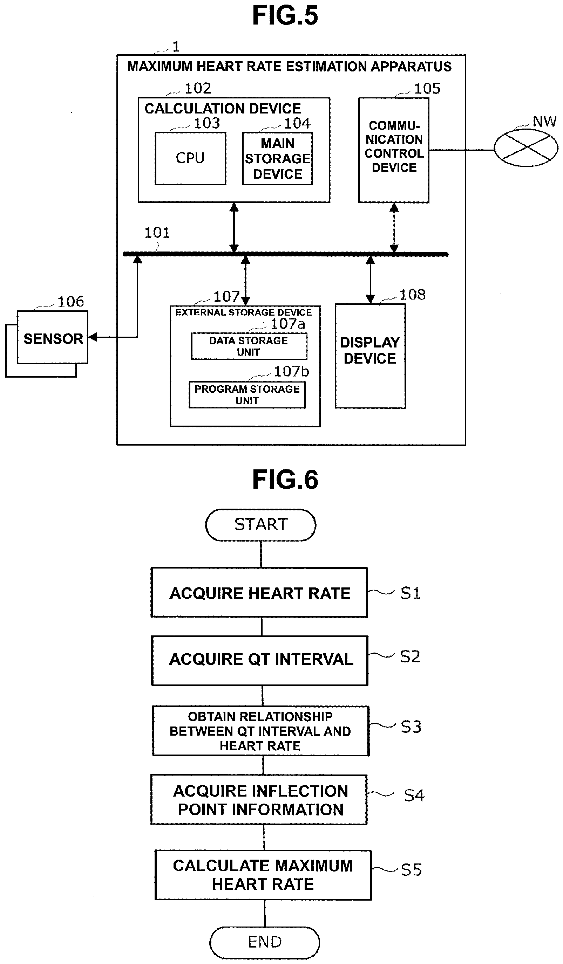

The hardware arrangement of the maximum heart rate estimation device 1 having the above-described functional arrangement will be described next with reference to a block diagram shown in Fig. 5.

As shown in Fig. 5, the maximum heart rate estimation device 1 can be implemented by a computer including a calculation device 102 with a CPU 103 and a main storage device 104, a communication control device 105, a sensor 106, an external storage device 107, and a display device 108, all of which are connected via a bus 101, and a program for controlling these hardware resources.

The CPU 103 and the main storage device 104 form the calculation device 102. A program used by the CPU 103 to perform various control and calculation operations is stored in advance in the main storage device 104. The calculation device 102 implements the functions of the maximum heart rate estimation device 1 including the estimation unit 13 shown in Fig. 4.

The communication control device 105 is a control device for connecting the maximum heart rate estimation device 1 and various external electronic devices by a communication network NW. The communication control device 105 may receive, via the communication network NW, the data of the heart rate and electrocardiograph waveform from the sensor 106 (to be described later) attached to the target person.

The sensor 106 is implemented by, for example, a biological sensor such as a heart rate meter and an electrocardiograph. The sensor 106 is attached to, for example, the chest or wrist of the target person for a period during which the target person does exercise, and measures the heart rate and the electrocardiographic waveform of the target person. For example, the sensor 106 attached to the chest measures the electrocardiographic waveform by an electrode (not shown), and detects heartbeats from a change of the electrocardiographic waveform, thereby measuring, as a heart rate, a heartbeat count per minute from an interval between the heartbeats.

The external storage device 107 is formed by a readable/writable storage medium and a driving device for reading/writing various kinds of information such as programs and data from/in the storage medium. For the external storage device 107, a hard disk or a semiconductor memory such as a flash memory can be used as a storage medium. The external storage device 107 can include a data storage unit 107a, a program storage unit 107b, and another storage device (not shown), for example, a storage device for backing up the programs and data stored in the external storage device 107.

The data storage unit 107a stores information concerning the electrocardiographic waveform and the heart rate of the target person measured by the sensor 106. The data storage unit 107a corresponds to the storage unit 12 shown in Fig. 4.

The program storage unit 107b stores various programs for executing processing necessary to estimate the maximum heart rate, such as processing of acquiring the heart rate and QT interval, inflection point processing, and maximum heart rate calculation processing, according to this embodiment.

The display device 108 forms the display screen of the maximum heart rate estimation device 1, and functions as the output unit 14. The display device 108 is implemented by a liquid crystal display or the like.

[Operation of Maximum Heart Rate Estimation Device]

The operation of the maximum heart rate estimation device 1 for executing the above-described maximum heart rate estimation method according to the present invention will be described next with reference to a flowchart shown in Fig. 6. First, a biological sensor (not shown) having the functions of the heart rate meter and electrocardiograph is attached to the chest or wrist of a target person, and the target person starts exercise like an incremental load test that gradually increases exercise intensity. The biological sensor measures the heart rate and electrocardiographic waveform of the target person for a period from when the target person starts exercise until the exercise intensity of the target person exceeds 40%.

The heart rate acquisition unit 111 acquires heart rate data while the target person does the exercise (step S1). Next, the QT interval acquisition unit 112 acquires electrocardiographic waveform data while the target person does the exercise, and acquires QT interval data from the electrocardiographic waveform data (step S2). Note that the QT interval acquisition unit 112 may adopt an arrangement of acquiring the value of a QT interval obtained by the external biological sensor.

Next, the inflection point processing unit 131 obtains the relationship between the acquired QT interval and heart rate (step S3). In the relationship between the QT interval and the heart rate of the target person, which has been obtained by the inflection point processing unit 131, the inflection point of the value of the QT interval is extracted, and a heart rate corresponding to the value of the QT interval is obtained (step S4).

Next, the maximum heart rate calculation unit 132 calculates the maximum exercise intensity of the target person using equation (1) above based on the heart rate at the inflection point of the measured value of the QT interval obtained in step S4 (step S5).

More specifically, the maximum heart rate calculation unit 132 uses, in equation (1), a value actually measured as the value of "resting heart rate", for example, 60 bpm. The maximum heart rate calculation unit 132 substitutes, as the value of "heart rate at inflection point", the measured value of the heart rate obtained in step S4. Furthermore, a value falling within the predetermined range of 35% to 45%, for example, 40% is used as the value of "exercise intensity at the inflection point" from the above-described experiment result.

Note that the output unit 14 outputs the calculated maximum exercise intensity of the target person.

As described above, according to the first embodiment, the maximum heart rate is estimated based on the heart rate of the target person at the inflection point of the QT interval around a portion where the exercise intensity (heart rate) is 40% using the relationship between the QT interval and the heart rate of the target person. Therefore, if exercise is done until the exercise intensity exceeds 40%, it is possible to estimate the maximum heart rate without requiring exercise to be done until the heart rate of the target person reaches the maximum heart rate. Thus, it is possible to reduce the load of the target person when obtaining the maximum heart rate.

Furthermore, the above-described first embodiment has explained the case in which the QT interval is acquired from the measured electrocardiographic waveform of the target person. However, an RT interval as the time from the start of an R wave to the end of a T wave included in the electrocardiographic waveform shown in Fig. 1 may be used as a feature amount of the electrocardiographic waveform, instead of the QT interval. It is possible to acquire a feature amount corresponding to the QT interval more easily using the RT interval.

[Second Embodiment]

The second embodiment of the present invention will be described next. Note that in the following description, the same reference numerals as those in the above-described first embodiment denote similar components and a repetitive description thereof will be omitted.

In the first embodiment, a heart rate corresponding to the inflection point of the value of the QT interval around a portion where the exercise intensity (heart rate) is about 40% is obtained based on the relationship between the QT interval and heart rate measured for the period during which the target person does exercise, thereby calculating the maximum heart rate of the target person. To the contrary, in the second embodiment, the height of a T wave included in the electrocardiographic waveform of a target person is used as a feature amount of the electrocardiographic waveform.

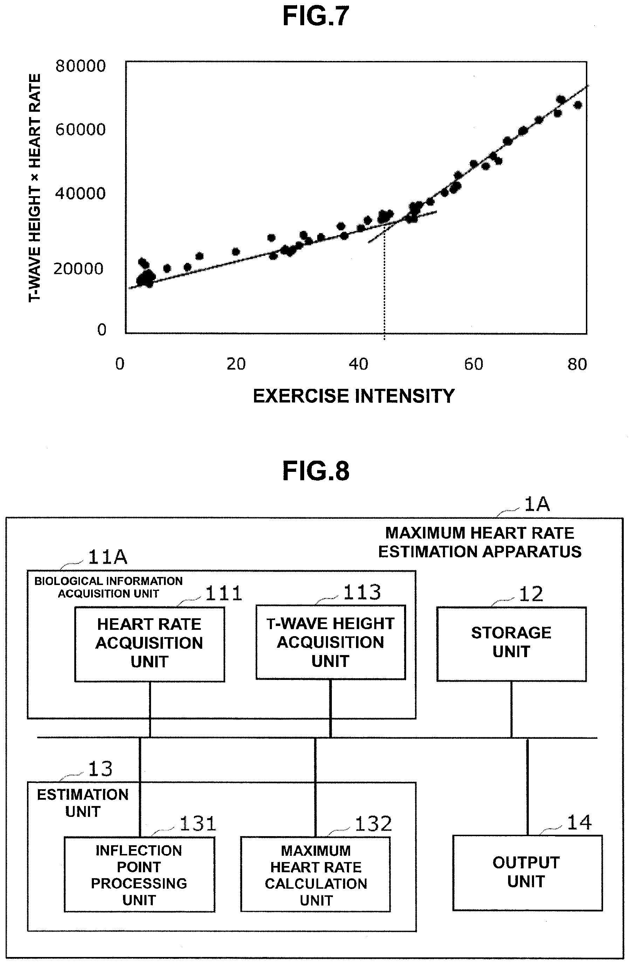

An overview of a maximum heart rate estimation device 1A according to the second embodiment will be described with reference to Fig. 7. In a graph shown in Fig. 7, the abscissa represents exercise intensity obtained by heart rate conversion, and the ordinate represents a value (T-wave height $\times$ heart rate) obtained by multiplying the height of a T wave in the electrocardiographic waveform of a target person by a heart rate.

As shown in Fig. 7, in "T-wave height of electrocardiographic waveform $\times$ heart rate" as well, there may be an inflection point around a portion where the exercise intensity (heart rate) is 40%. Therefore, it is possible to calculate the maximum heart rate of the target person using equation (1) above, similar to the first embodiment, by obtaining a measured heart rate corresponding to the inflection point.

With respect to the functional arrangement of the maximum heart rate estimation device 1A according to this embodiment, components different from those in the first embodiment will mainly be described next.

A biological information acquisition unit 11A includes a heart rate acquisition unit 111 and a T-wave height acquisition unit 113.

The T-wave height acquisition unit 113 acquires an electrocardiographic waveform from a biological sensor attached to a target person, and acquires data of a T-wave height included in the electrocardiographic waveform. The data of the T-wave height acquired by the T-wave height acquisition unit 113 is stored in a storage unit 12.

An inflection point processing unit 131 calculates "T-wave height $\times$ heart rate" by multiplying the value of the T-wave height acquired by the T-wave height acquisition unit 113 of the biological information acquisition unit 11A by the value of the heart rate of the target person acquired by a heart rate acquisition unit 111. Furthermore, the inflection point processing unit 131 obtains the relationship between "T-wave height $\times$ heart rate" and the measured value of the heart rate of the target person acquired by the heart rate acquisition unit 111.

The inflection point processing unit 131 finds the inflection point of the value of "T-wave height $\times$ heart rate" around a portion where the exercise intensity (heart rate) is 40% in the obtained relationship between the heart rate and "T-wave height $\times$ heart rate" of the target person. The inflection point processing unit 131 obtains a heart rate corresponding to the inflection point. The value of the obtained heart rate is stored in the storage unit 12.

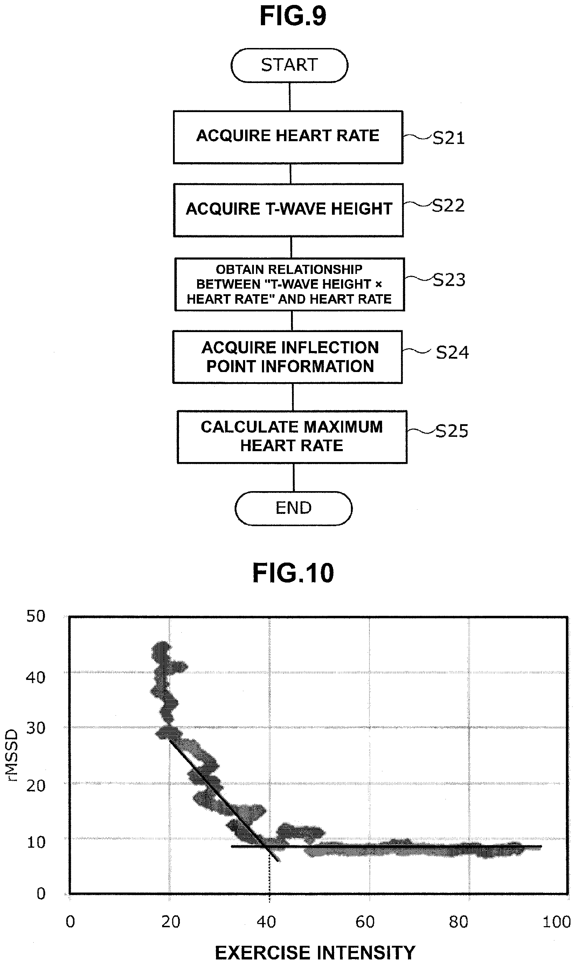

The operation of the maximum heart rate estimation device 1A having the above-described arrangement will be described next with reference to a flowchart shown in Fig. 9.

Similar to the first embodiment, first, a biological sensor (not shown) having the functions of a heart rate meter and electrocardiograph is attached to the chest or wrist of the target person, and the target person starts exercise like an incremental load test that gradually increases exercise intensity. The biological sensor measures the heart rate and electrocardiographic waveform of the target person for a period from when the target person starts the exercise until the exercise intensity of the exercise done by the target person exceeds 40%.

The heart rate acquisition unit 111 acquires heart rate data for a period during which the target person does the exercise (step S21). Next, the T-wave height acquisition unit 113 acquires T-wave height data from electrocardiographic waveform data for the period during which the target person does the exercise (step S22). Note that the T-wave height acquisition unit 113 may adopt an arrangement of acquiring T-wave height data obtained on the biological sensor side.

The inflection point processing unit 131 obtains the relationship between the acquired heart rate and "T-wave height $\times$ heart rate" (step S23). After that, the inflection point processing unit 131 extracts the inflection point of the value of "T-wave height $\times$ heart rate" in the obtained relationship between the heart rate and "T-wave height $\times$ heart rate", and obtains the value of a heart rate corresponding to the value of "T-wave height $\times$ heart rate" (step S24).

Next, a maximum heart rate calculation unit 132 calculates the maximum exercise intensity of the target person using equation (1) above based on the heart rate at the inflection point of the value of "T-wave height $\times$ heart rate" obtained in step S24 (step S25) .

More specifically, the maximum heart rate calculation unit 132 uses, in equation (1), a value actually measured as the value of "resting heart rate", for example, 60 bpm. The maximum heart rate calculation unit 132 substitutes, as the value of "heart rate at inflection point", the heart rate obtained in step S24. Furthermore, a predetermined value, for example, a value such as 40% falling within the range of 35% to 45% is used as the value of "exercise intensity at the inflection point" from the above-described experiment result.

Note that an output unit 14 outputs the calculated maximum exercise intensity of the target person.

As described above, according to the second embodiment, the maximum heart rate is estimated based on the heart rate of the target person at the inflection point of "T-wave height $\times$ heart rate" around a portion where the exercise intensity (heart rate) is 40% using the relationship between the heart rate and "T-wave height $\times$ heart rate" of the target person. Therefore, even when the T-wave height is used as a feature amount of the electrocardiographic waveform, if exercise is done until the exercise intensity exceeds 40%, it is possible to estimate the maximum heart rate without requiring exercise to be done until the heart rate of the target person reaches the maximum heart rate. Thus, it is possible to reduce the load of the target person when obtaining the maximum heart rate.

[Third Embodiment]

The third embodiment of the present invention will be described next. Note that in the following description, the same reference numerals as those in the above-described first and second embodiments denote similar components and a repetitive description thereof will be omitted.

In the first and second embodiments, based on the relationship between the heart rate measured for the period during which the target person does the exercise and a feature amount such as the QT interval or T-wave height observed in the electrocardiographic waveform, the maximum heart rate of the target person is calculated from the heart rate corresponding to the inflection point of the feature amount around a portion where the exercise intensity is about 40%. To the contrary, in the third embodiment, rMSSD (Root Mean Square of Successive Differences) as a heart rate variability parameter known as an autonomic nerve index is used as a feature amount.

First, an overview of a maximum heart rate estimation method according to the third embodiment will be described with reference to Fig. 10.

Fig. 10 is a graph for explaining the relationship between rMSSD and exercise intensity. The ordinate represents rMSSD and the abscissa represents exercise intensity obtained by heart rate conversion.

Note that rMSSD is the root mean square of successive differences between neighboring R-R intervals in an electrocardiographic waveform, and is known as an index concerning autonomic nerves. As is apparent from Fig. 10, in the relationship between rMSSD and the exercise intensity as well, there is the inflection point of the value of rMSSD around a portion where the exercise intensity is 40%.

Therefore, the maximum heart rate of the target person is calculated by equation (1) above using the rMSSD value as the feature amount of the electrocardiographic waveform.

Next, with respect to the functional arrangement of a maximum heart rate estimation device 1B for executing a maximum heart rate estimation method according to this embodiment, components different from those in the first and second embodiments will mainly be described.

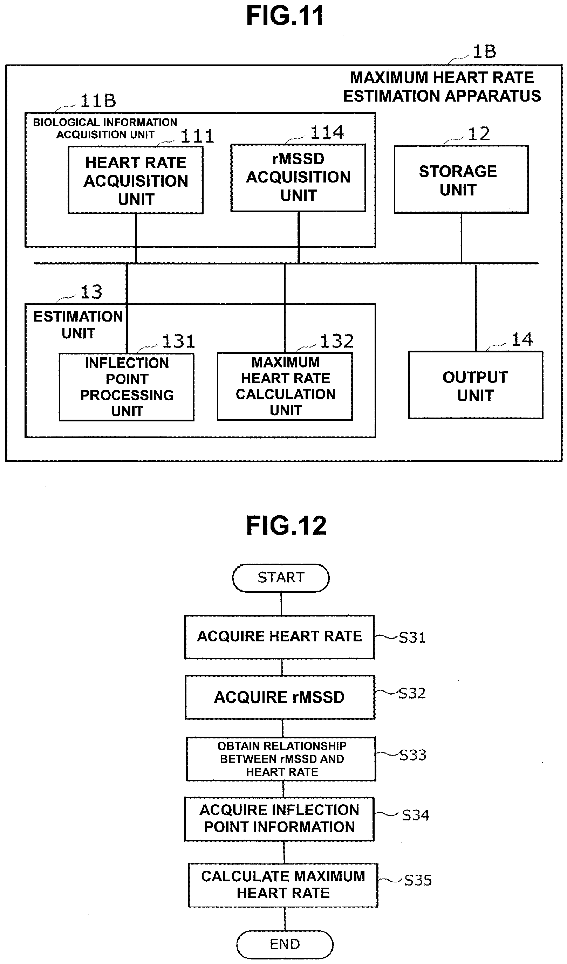

As shown in Fig. 11, a biological information acquisition unit 11B includes a heart rate acquisition unit 111 and an rMSSD acquisition unit 114.

The rMSSD acquisition unit 114 acquires an electrocardiographic waveform from a biological sensor attached to a target person, and acquires data of neighboring R-R intervals included in the electrocardiographic waveform. The rMSSD acquisition unit 114 calculates rMSSD based on the acquired data indicating the R-R intervals. The rMSSD value of the target person calculated by the rMSSD acquisition unit 114 is stored in a storage unit 12.

An inflection point processing unit 131 obtains the relationship between the heart rate of the target person acquired by the heart rate acquisition unit 111 and rMSSD of the target person acquired by the rMSSD acquisition unit 114. The inflection point processing unit 131 extracts the inflection point of the rMSSD value in the obtained relationship between the heart rate and rMSSD of the target person. The inflection point processing unit 131 obtains a heart rate corresponding to the inflection point. The value of the obtained heart rate is stored in the storage unit 12.

The operation of the maximum heart rate estimation device 1B having the above-described arrangement will be described next with reference to a flowchart shown in Fig. 12.

Similar to the first and second embodiments, first, the heart rate acquisition unit 111 acquires the measured value of a heart rate for a period from when the target person does exercise like an incremental load test that gradually increases exercise intensity until the exercise intensity exceeds 40% (step S31).

Next, the rMSSD acquisition unit 114 acquires data indicating the R-R intervals from an electrocardiographic waveform sent from the biological sensor attached to the target person, and calculates rMSSD (step S32). Note that the rMSSD acquisition unit 114 may adopt an arrangement of acquiring an rMSSD value calculated by the external biological sensor.

Next, the inflection point processing unit 131 obtains the relationship between the heart rate of the target person acquired by the heart rate acquisition unit 111 and rMSSD of the target person acquired by the rMSSD acquisition unit 114 (step S33).

After that, the inflection point processing unit 131 finds the inflection point of the rMSSD value in the obtained relationship between the heart rate and rMSSD of the target person, and obtains the value of a heart rate corresponding to the rMSSD value (step S34).

Next, a maximum heart rate calculation unit 132 calculates the maximum exercise intensity of the target person using equation (1) above based on the heart rate at the inflection point of the rMSSD value obtained in step S34 (step S35).

More specifically, the maximum heart rate calculation unit 132 uses, in equation (1), a value actually measured as the value of "resting heart rate", for example, 60 bpm. The maximum heart rate calculation unit 132 substitutes, as the value of "heart rate at inflection point", the heart rate obtained in step S34. Furthermore, a preset value, for example, a value such as 40% falling within the range of 35% to 45% is used as the value of "exercise intensity at the inflection point" from the above-described experiment result.

Note that an output unit 14 outputs the calculated maximum exercise intensity of the target person.

As described above, according to the third embodiment, the maximum heart rate is estimated based on the heart rate of the target person at the inflection point of rMSSD around a portion where the exercise intensity is 40% using the relationship between the heart rate and rMSSD of the target person. Therefore, even when rMSSD is used as a feature amount in the electrocardiographic waveform, if exercise is done until the exercise intensity exceeds 40%, it is possible to estimate the maximum heart rate without requiring exercise to be done until the heart rate of the target person reaches the maximum heart rate. Thus, it is possible to reduce the load of the target person when obtaining the maximum heart rate.

This embodiment estimates the maximum heart rate of the target person by paying attention to rMSSD serving as the autonomic nerve index but can use another autonomic nerve index instead of rMSSD. Therefore, it is possible to estimate the maximum heart rate with higher flexibility. Other examples of the autonomic nerve index are an LF value, HF value, and LF/HF ratio in the frequency domain and SDNN (Standard Deviation of the NN intervals), NN50, and pNN50 in the time domain (see, for example, non-patent literature 3).

The embodiments in the maximum heart rate estimation method and the maximum heart rate estimation device according to the present invention have been described above. However, the present invention is not limited to the above-described embodiments, and various modifications conceivable by those skilled in the art can be made within the scope of the invention described in the claims.

Note that each of the above-described embodiments has explained the case in which the target person does exercise like an incremental load test and data concerning a heart rate and an electrocardiographic waveform for an exercise period is acquired. However, the exercise done by the target person need not be managed exercise such as the incremental load test. The maximum heart rate may be estimated by acquiring the relationship between a heart rate and a feature amount such as a QT interval from arbitrary exercise data and obtaining a heart rate at an inflection point. Explanation of the Reference Numerals and Signs

1, 1A, 1B...maximum heart rate estimation device, 11, 11A, 11B...biological information acquisition unit, 12...storage unit, 13...estimation unit, 14...output unit, 111...heart rate acquisition unit, 112...QT interval acquisition unit, 113...T-wave height acquisition unit, 114...rMSSD acquisition unit, 131...inflection point processing unit, 132...maximum heart rate calculation unit, 101...bus, 102...calculation device, 103...CPU, 104...main storage device, 105...communication control device, 106...sensor, 107...external storage device, 107a...data storage unit, 107b...program storage unit, 108...display device, NW...communication network

**Claims**

1. A maximum heart rate estimation method comprising:

   a first acquisition step (S1, S21, S31) of acquiring, by a heart rate acquisition unit (111), a heart rate of a target person who does exercise in an incremental load test that gradually increases exercise intensity;
   a second acquisition step of acquiring, by a feature amount acquisition unit (112), an electrocardiographic waveform of the target person who does the exercise in an incremental load test that gradually increases exercise intensity;
   a third acquisition step (S2, S22, S32) of acquiring, by the feature amount acquisition unit (112), a predetermined feature amount from the acquired electrocardiographic waveform, the predetermined feature amount being a QT interval, an RT interval, a height of a T wave, Root Mean Square of Successive Differences, an LF value, an HF value, an LF/HF ratio, Standard Deviation of the NN intervals, NN50, or pNN50;
   a step (S3) of obtaining, by an inflection point processing unit (131), a relationship between the predetermined feature amount and the heart rate, an extraction step (S4, S24, S34) of extracting, by the inflection point processing unit (131), an inflection point of the predetermined feature amount in the relationship between the predetermined feature amount and the heart rate, and obtaining a heart rate corresponding to the value of the feature amount at the inflection point, wherein the heart rate acquired in the first acquisition step (S1, S21, S31) corresponding to the value of the feature amount at the inflection point is set to correspond to an exercise intensity falling within a range of 35% to 45%; and
   an estimation step (S5, S25, S35) of estimating, by a maximum heart rate calculation unit (132), a maximum heart rate of the target person based on the relationship between the predetermined feature amount and the acquired heart rate,
   **characterized in that**
   the relationship between the feature amount acquired in the second acquisition step (S2, S22, S32) and the heart rate acquired in the first acquisition step (S1, S21, S31) contains the inflection point;
   in the estimation step, the maximum heart rate of the target person is estimated based on a heart rate corresponding to the value of the feature amount at the inflection point in a change of the predetermined feature amount with respect to the acquired heart rate, and wherein the estimation step includes a step of estimating the maximum heart rate using the equation: "maximum heart rate = resting heart rate + 100 × (heart rate at inflection point - resting heart rate)/exercise intensity at inflection point", where "resting heart rate" is a heart rate measured in advance at the time of rest, "heart rate at inflection point" is the heart rate corresponding to the value of the feature amount at the inflection point, and "exercise intensity at inflection point" is the preset value being within a range of 35% to 45%.

2. A maximum heart rate estimation device (1, 1A, 1B) comprising:

a heart rate acquisition unit (111) configured to acquire a heart rate of a target person who does exercise in an incremental load test that gradually increases exercise intensity;
a feature amount acquisition unit (112, 113, 114) configured to acquire an electrocardiographic waveform of the target person who does the exercise in an incremental load test that gradually increases exercise intensity, and acquire a predetermined feature amount from the electrocardiographic waveform, the predetermined feature amount being a QT interval, an RT interval, a height of a T wave, Root Mean Square of Successive Differences, an LF value, an HF value, an LF/HF ratio, Standard Deviation of the NN intervals, NN50, or pNN50;
an inflection point processing unit (131) configured to extract an inflection point of the predetermined feature amount in the relationship between the predetermined feature amount and the heart rate, and to obtain a heart rate corresponding to the value of the feature amount at the inflection point, wherein the heart rate acquired by the heart rate acquisition unit corresponding to the value of the feature amount at the inflection point is set to correspond to an exercise intensity falling within a range of 35% to 45%; and
an estimation unit (13) configured to estimate a maximum heart rate of the target person based on the relationship between the acquired heart rate and the predetermined feature amount, wherein

the heart rate acquisition unit (111) and the feature amount acquisition unit (112, 113, 114) are configured to acquire the heart rate and respectively the predetermined feature amount such that the relationship between the predetermined feature amount and the heart rate contains an inflection point;
the estimation unit (13) is configured to estimate the maximum heart rate of the target person based on a heart rate corresponding to the value of the feature amount at the inflection point in a change of the predetermined feature amount with respect to the acquired heart rate, and wherein the estimation unit (13) is configured to estimate the maximum heart rate using the equation:

"maximum heart rate = resting heart rate + 100 × (heart rate at inflection point - resting heart rate)/exercise intensity at inflection point",

inflection point - resting heart rate)/exercise intensity at inflection point", where "resting heart rate" is a heart rate measured in advance at the time of rest, "heart rate at inflection point" is a heart rate corresponding to the value of the feature amount at the inflection point, and "exercise intensity at inflection point" is the preset value being within a range of 35% to 45%.

**Patentansprüche**

1. Verfahren zum Schätzen der maximalen Herzschlagrate, umfassend:

einen ersten Erfassungsschritt (S1, S21, S31) des Erfassens, durch eine Herzschlagraten-Erfassungseinheit (111), einer Herzschlagrate einer Zielperson, die ein Training in einem inkrementellen Belastungstest durchführt, bei dem die Trainingsintensität graduell erhöht wird;
einen zweiten Erfassungsschritt des Erfassens einer elektrokardiographischen Messkurve der Zielperson, die das Training in einem inkrementellen Belastungstest durchführt, bei dem die Trainingsintensität graduell erhöht wird, durch eine Merkmalswert-Erfassungseinheit (112);
einen dritten Erfassungsschritt (S2, S22, S32) des Erfassens eines vorbestimmten Merkmalswertes aus der erfassten elektrokardiographischen Messkurve durch die Merkmalswert-Erfassungseinheit (112), wobei der vorbestimmte Merkmalswert ein QT-Intervall, ein RT-Intervall, eine Höhe einer T-Welle, ein mittleres Wurzelquadrat aufeinanderfolgender Differenzen, ein LF-Wert, ein HF-Wert, ein LF/HF-Verhältnis, eine Standardabweichung der NN-Intervalle, NN50, oder pNN50 ist;
einen Schritt (S3) des Erhaltens, durch eine Flexionspunkt-Verarbeitungseinheit (131), einer Beziehung zwischen dem vorbestimmten Merkmalswert und der Herzschlagrate, einen Extraktions-Schritt (S4, S24, S34) des Extrahierens, durch die Flexionspunkt-Verarbeitungseinheit (131), eines Flexionspunktes des vorbestimmten Merkmalswertes in der Beziehung zwischen dem vorbestimmten Merkmalswert und der Herzschlagrate, und Erhalten einer Herzschlagrate, die dem Wert des Merkmalswertes an dem Flexionspunkt entspricht, wobei die in dem ersten Erfassungsschritt (S1, S21, S31) erfasste Herzschlagrate, die dem Wert des Merkmalswertes an dem Flexionspunkt entspricht, festgesetzt wird, einer Trainingsintensität zu entsprechen, die in einen Bereich von 35% bis 45% fällt; und

einen Schätzschritt (S5, S25, S35) zum Schätzen einer maximalen Herzschlagrate der Zielperson durch eine Herzschlagraten-Berechnungseinheit (132) auf der Grundlage der Beziehung zwischen dem vorgegebenen Merkmalswert und der erfassten Herzschlagrate,

**dadurch gekennzeichnet, dass**

die Beziehung zwischen dem im zweiten Erfassungsschritt (S2, S22, S32) erfassten Merkmalswert und der im ersten Erfassungsschritt (S1, S21, S31) erfassten Herzschlagrate den Flexionspunkt enthält;

im Schätzschritt die maximale Herzschlagrate der Zielperson auf der Grundlage einer Herzschlagrate geschätzt wird, die dem Wert des Merkmalswerts am Flexionspunkt in einer Änderung des vorbestimmten Merkmalswerts gegenüber der erfassten Herzschlagrate entspricht, und wobei der Schätzschritt einen Schritt des Schätzens der maximalen Herzschlagrate unter Verwendung der Gleichung "maximale Herzschlagrate = Ruheherzschlagrate + 100 × (Herzschlagrate am Flexionspunkt - Ruheherzschlagrate)/Trainingsintensität am Flexionspunkt" umfasst, wobei "Ruheherzfrequenz" eine Herzschlagrate ist, die im Voraus zu einem Ruhezeitpunkt gemessen wird, "Herzfrequenz am Flexionspunkt" die Herzfrequenz ist, die dem Wert des Merkmalswertes am Flexionspunkt entspricht, und "Trainingsintensität am Flexionspunkt" der vorab festgelegte Wert ist, der in einem Bereich von 35% bis 45% liegt.

2. Vorrichtung zur Schätzung der maximalen Herzschlagrate (1, 1A, 1B), umfassend:

eine Herzschlagraten-Erfassungseinheit (111), die so konfiguriert ist, dass sie eine Herzschlagrate einer Zielperson erfasst, die ein Training in einem inkrementellen Belastungstest durchführt, bei dem die Trainingsintensität graduell erhöht wird;

eine Merkmalswert-Erfassungseinheit (112, 113, 114), die so konfiguriert ist, dass sie eine elektrokardiographische Messkurve der Zielperson erfasst, die das Training in einem inkrementellen Belastungstest durchführt, bei dem die Trainingsintensität graduell erhöht wird, und, dass sie einen vorbestimmten Merkmalswert aus der elektrokardiographischen Messkurve erfasst, wobei der vorbestimmte Merkmalswert ein QT-Intervall, ein RT-Intervall, eine Höhe einer T-Welle, der mittlere quadratische Wert aufeinanderfolgender Differenzen, ein LF-Wert, ein HF-Wert, ein LF/HF-Verhältnis, die Standardabweichung der NN-Intervalle, NN50 oder pNN50 ist;

eine Flexionspunkt-Verarbeitungseinheit (131), die konfiguriert ist, um einen Flexionspunkt des vorbestimmten Merkmalswerts in der Beziehung zwischen dem vorbestimmten Merkmalswert und der Herzschlagrate zu extrahieren und eine Herzschlagrate zu erhalten, die dem Wert des Merkmalswerts am Flexionspunkt entspricht, wobei die von der Herzschlagraten-Erfassungseinheit erfasste Herzschlagrate, die dem Wert des Merkmalswerts am Flexionspunkt entspricht, festgesetzt wird, einer Trainingsintensität zu entsprechen, die in einem Bereich von 35 % bis 45 % liegt; und

eine Schätzeinheit (13), die konfiguriert ist, um eine maximale Herzschlagrate der Zielperson auf der Grundlage der Beziehung zwischen der erfassten Herzschlagrate und dem vorbestimmten Merkmalswert zu schätzen, wobei

die Herzschlagraten-Erfassungseinheit (111) und die Merkmalswert-Erfassungseinheit (112, 113, 114) so konfiguriert sind, dass sie die Herzschlagrate bzw. den vorgegebenen Merkmalswert so erfassen, so dass die Beziehung zwischen dem vorgegebenen Merkmalswert und der Herzschlagrate einen Flexionspunkt enthält;

die Schätzeinheit (13) so konfiguriert ist, dass sie die maximale Herzschlagrate der Zielperson auf der Grundlage einer Herzschlagrate schätzt, die dem Wert des Merkmalswerts am Flexionspunkt in einer Änderung des vorbestimmten Merkmalswerts gegenüber der erfassten Herzschlagrate entspricht, und wobei die Schätzeinheit (13) so konfiguriert ist, dass sie die maximale Herzschlagrate unter Verwendung der Gleichung "maximale Herzschlagrate = Ruheherzschlagrate + 100 × (Herzschlagrate am Flexionspunkt - Ruheherzschlagrate)/Trainingsintensität am Flexionspunkt" schätzt, wobei "Ruheherzfrequenz" eine Herzschlagrate ist, die im Voraus zu einem Ruhezeitpunkt gemessen wird, "Herzfrequenz am Flexionspunkt" die Herzfrequenz ist, die dem Wert des Merkmalswertes am Flexionspunkt entspricht, und "Trainingsintensität am Flexionspunkt" der vorab festgelegte Wert ist, der in einem Bereich von 35% bis 45% liegt.

## EP 3 777 668 B1

**Revendications**

1.  Procédé d'estimation de fréquence cardiaque maximale comprenant :

    une première étape d'acquisition (S1, S21, S31) consistant à acquérir, par une unité d'acquisition de fréquence cardiaque (111), une fréquence cardiaque d'une personne cible qui fait un exercice dans un test de charge incrémentielle qui augmente progressivement l'intensité de l'exercice ;
    une deuxième étape d'acquisition consistant à acquérir, par une unité d'acquisition de quantité de caractéristique (112), une forme d'onde électrocardiographique de la personne cible qui fait l'exercice dans un test de charge incrémentielle qui augmente progressivement l'intensité de l'exercice ;
    une troisième étape d'acquisition (S2, S22, S32) consistant à acquérir, par l'unité d'acquisition de quantité de caractéristique (112), une quantité de caractéristique prédéterminée à partir de la forme d'onde électrocardiographique acquise, la quantité de caractéristique prédéterminée étant un intervalle QT, un intervalle RT, une hauteur d'une onde T, la moyenne quadratique de différences successives, une valeur LF, une valeur HF, un rapport LF/HF, un écart-type des intervalles NN, NN50 ou pNN50 ;
    une étape (S3) consistant à obtenir, par une unité de traitement de point d'inflexion (131), une relation entre la quantité de caractéristique prédéterminée et la fréquence cardiaque, une étape d'extraction (S4, S24, S34) consistant à extraire, par l'unité de traitement de point d'inflexion (131), un point d'inflexion de la quantité de caractéristique prédéterminée dans la relation entre la quantité de caractéristique prédéterminée et la fréquence cardiaque, et à obtenir une fréquence cardiaque correspondant à la valeur de la quantité de caractéristique au point d'inflexion, dans lequel la fréquence cardiaque acquise lors de la première étape d'acquisition (S1, S21, S31) correspondant à la valeur de la quantité de caractéristique au point d'inflexion est définie pour correspondre à une intensité d'exercice se trouvant dans une plage de 35 % à 45 % ; et
    une étape d'estimation (S5, S25, S35) consistant à estimer, par une unité de calcul de fréquence cardiaque (132), une fréquence cardiaque maximale de la personne cible sur la base de la relation entre la quantité de caractéristique prédéterminée et la fréquence cardiaque acquise,
    **caractérisé en ce que**
    la relation entre la quantité de caractéristique acquise lors de la deuxième étape d'acquisition (S2, S22, S32) et la fréquence cardiaque acquise lors de la première étape d'acquisition (S1, S21, S31) contient le point d'inflexion ;
    lors de l'étape d'estimation, la fréquence cardiaque maximale de la personne cible est estimée sur la base d'une fréquence cardiaque correspondant à la valeur de la quantité de caractéristique au point d'inflexion lors d'un changement de la quantité de caractéristique prédéterminée par rapport à la fréquence cardiaque acquise, et dans lequel l'étape d'estimation comporte une étape consistant à estimer la fréquence cardiaque maximale à l'aide de l'équation :
    « fréquence cardiaque maximale = fréquence cardiaque au repos + 100 $\times$ (fréquence cardiaque au point d'inflexion - fréquence cardiaque au repos)/intensité de l'exercice au point d'inflexion », où « fréquence cardiaque au repos » est une fréquence cardiaque mesurée au préalable au moment du repos, « fréquence cardiaque au point d'inflexion » est la fréquence cardiaque correspondant à la valeur de la quantité de caractéristique au point d'inflexion, et « intensité de l'exercice au point d'inflexion » est la valeur prédéfinie se trouvant dans une plage de 35 % à 45 %.

2.  Dispositif d'estimation de fréquence cardiaque maximale (1, 1A, 1B) comprenant :

    une unité d'acquisition de fréquence cardiaque (111) configurée pour acquérir une fréquence cardiaque d'une personne cible qui fait un exercice dans un test de charge incrémentielle qui augmente progressivement l'intensité de l'exercice ;
    une unité d'acquisition de quantité de caractéristique (112, 113, 114) configurée pour acquérir une forme d'onde électrocardiographique de la personne cible qui fait l'exercice dans un test de charge incrémentielle qui augmente progressivement l'intensité de l'exercice, et acquérir une quantité de caractéristique prédéterminée à partir de la forme d'onde électrocardiographique, la quantité de caractéristique prédéterminée étant un intervalle QT, un intervalle RT, une hauteur d'une onde T, la moyenne quadratique de différences successives, une valeur LF, une valeur HF, un rapport LF/HF, un écart-type des intervalles NN, NN50 ou pNN50 ;
    une unité de traitement de point d'inflexion (131) configurée pour extraire un point d'inflexion de la quantité de caractéristique prédéterminée dans la relation entre la quantité de caractéristique prédéterminée et la fréquence cardiaque, et pour obtenir une fréquence cardiaque correspondant à la valeur de la quantité de caractéristique au point d'inflexion, dans lequel la fréquence cardiaque acquise par l'unité d'acquisition de fréquence cardiaque correspondant à la valeur de la quantité de caractéristique au point d'inflexion est définie pour correspondre à une intensité d'exercice se trouvant dans une plage de 35 % à 45 % ; et

une unité d'estimation (13) configurée pour estimer une fréquence cardiaque maximale de la personne cible sur la base de la relation entre la fréquence cardiaque acquise et la quantité de caractéristique prédéterminée, dans lequel

l'unité d'acquisition de fréquence cardiaque (111) et l'unité d'acquisition de quantité de caractéristique (112, 113, 114) sont configurées pour acquérir la fréquence cardiaque et respectivement la quantité de caractéristique prédéterminée de telle sorte que la relation entre la quantité de caractéristique prédéterminée et la fréquence cardiaque contient un point d'inflexion ;

l'unité d'estimation (13) est configurée pour estimer la fréquence cardiaque maximale de la personne cible sur la base d'une fréquence cardiaque correspondant à la valeur de la quantité de caractéristique au point d'inflexion lors d'un changement de la quantité de caractéristique prédéterminée par rapport à la fréquence cardiaque acquise, et dans lequel l'unité d'estimation (13) est configurée pour estimer la fréquence cardiaque maximale à l'aide de l'équation :

« fréquence cardiaque maximale = fréquence cardiaque au repos + 100 × (fréquence cardiaque au point d'inflexion - fréquence cardiaque au repos)/intensité de l'exercice au point d'inflexion », où « fréquence cardiaque au repos » est une fréquence cardiaque mesurée au préalable au moment du repos, « fréquence cardiaque au point d'inflexion » est une fréquence cardiaque correspondant à la valeur de la quantité de caractéristique au point d'inflexion, et « intensité de l'exercice au point d'inflexion » est la valeur prédéfinie se trouvant dans une plage de 35 % à 45 %.

# FIG.1

# FIG.2

EXERCISE INTENSITY

# FIG.3

# FIG.4

# FIG.5

# FIG.6

# FIG.7

# FIG.8

# FIG.9

```
        ┌─────────────┐
        │    START    │
        └─────────────┘
               │
   ┌───────────────────────┐
   │   ACQUIRE HEART RATE   │─── S21
   └───────────────────────┘
               │
   ┌───────────────────────┐
   │  ACQUIRE T-WAVE HEIGHT │─── S22
   └───────────────────────┘
               │
   ┌───────────────────────┐
   │  OBTAIN RELATIONSHIP   │
   │ BETWEEN "T-WAVE HEIGHT ×│─── S23
   │ HEART RATE" AND HEART RATE│
   └───────────────────────┘
               │
   ┌───────────────────────┐
   │   ACQUIRE INFLECTION   │─── S24
   │    POINT INFORMATION   │
   └───────────────────────┘
               │
   ┌───────────────────────┐
   │   CALCULATE MAXIMUM    │─── S25
   │      HEART RATE        │
   └───────────────────────┘
               │
        ┌─────────────┐
        │     END     │
        └─────────────┘
```

# FIG.10

# FIG.11

1B

MAXIMUM HEART RATE
ESTIMATION APPARATUS

11B

BIOLOGICAL INFORMATION
ACQUISITION UNIT    111

| HEART RATE ACQUISITION UNIT | rMSSD ACQUISITION UNIT | STORAGE UNIT |

114

12

13

ESTIMATION UNIT

131

132

14

| INFLECTION POINT PROCESSING UNIT | MAXIMUM HEART RATE CALCULATION UNIT | OUTPUT UNIT |

# FIG.12

START

| ACQUIRE HEART RATE | S31 |

| ACQUIRE rMSSD | S32 |

| OBTAIN RELATIONSHIP BETWEEN rMSSD AND HEART RATE | S33 |

| ACQUIRE INFLECTION POINT INFORMATION | S34 |

| CALCULATE MAXIMUM HEART RATE | S35 |

END

19

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 2004147850 A1
- US 8755872 B1
- US 2017172513 A1
- US 4622980 A

### Non-patent literature cited in the description

- **LAURA KARAVIRTA et al.** Estimation of maximal heart rate using the relationship between heart rate variability and exercise intensity' in 40-67 years old men. *European Journal of Applied Physiology,* vol. 103 (1), 25-32
- **JINHUA SHE ; HITOSHI NAKAMURA ; KOJI MAKINO ; HIROSHI HASHIMOTO.** Selection of Suitable Maximum-heart-rate Formulas for Use with Karvonen Formula to Calculate Exercise Intensity. *International Journal of Automation and Computing,* February 2015, vol. 12 (1), 62-69
- **SHAFFER, FRED ; J.P.GINSBERG.** An overview of heart rate variability metrics and norms. *Frontiers in public health,* 2017, vol. 5, 258
- **HIDEAKI SENJU.** Chapter 3 Exercise and Change in Cardiopulmonary Function (Part I Regional Wellness). *Health Promotion from Life/Region, Nagasaki University Extension Course Series,* 05 March 1995, vol. 7, 31-39, <http://hdl.handle.net/10069/6315>